# EUROPEAN PATENT SPECIFICATION

(11) **EP 1 666 584 B1**
(45) Date of publication and mention of the grant of the patent: **29.12.2010**
(21) Application number: 04771982.8
(22) Date of filing: 20.08.2004
(51) Int. Cl.: C12N 1/15, C12N 15/00

(54) **METHOD OF BREEDING LIPID-PRODUCING FUNGUS**
VERFAHREN ZUR ZÜCHTUNG EINES LIPIDPRODUZIERENDEN PILZES
PROCEDE DE CULTURE D'UN CHAMPIGNON PRODUCTEUR DE LIPIDES

(30) Priority: 22.08.2003 JP 2003299345
(43) Date of publication of application: 07.06.2006
(73) Proprietor: Suntory Holdings Limited, Kita-ku, Osaka-shi Osaka 530-8203 (JP)
(72) Inventor: OCHIAI, Misa, Mishima-gun, Osaka 618-0001 (JP); KAWASHIMA, Hiroshi, Osaka 569-1121 (JP); SHIMIZU, Sakayu, Kyoto-shi, Kyoto 616-8212 (JP)
(74) Representative: Bentz, Jean-Paul
(86) International application number: PCT/JP2004/012021
(87) International publication number: WO 2005/019437

(56) References cited:
- JP-A- 7 143 894
- JP-A- 2000 069 987
- JP-A- 2000 125 859
- JP-A- 2002 510 498
- JP-A- 2002 516 116
- MACKENZIE D A ET AL: "Isolation and use of a homologous histone H4 promoter and a ribosomal DNA region in a transformation vector for the oil-producing fungus Mortierella alpina" APPLIED AND ENVIRONMENTAL MICROBIOLOGY, WASHINGTON,DC, US, vol. 66, no. 11, November 2000 (2000-11), pages 4655-4661, XP002291019 ISSN: 0099-2240
- DATABASE BIOSIS [Online] BIOSCIENCES INFORMATION SERVICE, PHILADELPHIA, PA, US; 1997, GONZALEZ-HERNANDEZ G A ET AL: "Biolistic transformation of Mucor circinelloides" XP002404905 Database accession no. PREV199799753376 & MYCOLOGICAL RESEARCH, vol. 101, no. 8, 1997, pages 953-956, ISSN: 0953-7562
- HERZOG R W ET AL: "A comparative study on the transformation of Aspergillus nidulans by microprojectile bombardment of conidia and a more conventional procedure using protoplasts treated with polyethyleneglycol" APPLIED MICROBIOLOGY AND BIOTECHNOLOGY, vol. 45, no. 3, 1996, pages 333-337, XP002403923 ISSN: 0175-7598
- ARMALEO DANIELE ET AL: "Biolistic nuclear transformation of Saccharomyces cerevisiae and other fungi" CURRENT GENETICS, vol. 17, no. 2, 1990, pages 97-103, XP002403924 ISSN: 0172-8083
- TAKENO SEIKI ET AL: "Establishment of an overall transformation system for an oil-producing filamentous fungus, Mortierella alpina 1S-4" APPLIED MICROBIOLOGY AND BIOTECHNOLOGY, vol. 65, no. 4, September 2004 (2004-09), pages 419-425, XP002403925 ISSN: 0175-7598
- APPEL K F ET AL: "A multicopy vector system for genetic studies in Mucor circinelloides and other zygomycetes" MGG MOLECULAR GENETICS AND GENOMICS, vol. 271, no. 5, June 2004 (2004-06), pages 595-602, XP002403926 ISSN: 1617-4615

## Description

### TECHNICAL FIELD

The present invention relates to a method for breeding a lipid-producing fungus, especially, a method for breeding a lipid-producing fungus belonging to *Mortierella* spp., the method being such that the breeding is carried out by transformation, but not selection using antibiotic.

### BACKGROUND ART

Techniques (fermentation techniques in a broad sense) for producing useful compounds by microorganism's metabolism have been developed and reduced in practice. Lipid-producing fungi capable of producing a lipid in a large quantity by its metabolism are some concrete examples. Fungi belonging to *Mortierella* spp. such as *Mortierella alpina* are typical lipid-producing fungi. *Mortierella* spp., which are known to produce polyunsaturated fatty acid (PUFA) such as arachidonic acid, are industrially useful fungi (e.g., see Japanese Patent Publication, *Tokukouhei*, No. 7-34752 (published on April 19, 1995) corresponding to Unexamined Japanese Patent Publication, Tokukaisho, No. 63-44891, (published on February 25, 1988)).

Breeding (i.e., breed improvement) for improving their genetic properties has been carried out for various useful organisms including microorganisms such as the lipid-producing fungi and the like. Breeding is very important especially in the fermentation technology in order to attain better efficiency, lower cost, and the like in microbial production of a compound.

The breeding method basically includes the step of creating a population including genetic variation (hereinafter, this step is referred to as the population creating step, for the sake of easy explanation), and the step of selecting, out of the population, a variety having a desirable property (hereinafter, this step is referred to as the selecting step, for the sake of easy explanation). Various processes can be adopted as the population creating step and selecting step depending on which kind of a useful organism is to be bred. For microorganisms such as the lipid-producing fungus and the like, (1) mutagen treating process or (2) transformation process is mainly adopted as the population creating step.

### (1) Mutagen treating process

In the population creation step adopting the mutagen treating process, the population is created by inducing mutation in the microorganism by various methods. The mutagenesis causes many kinds of mutations randomly. Therefore, even if a variety (strain) showing a targeted trait is obtained in the selecting step, there is a possibility that the variety would have an unexpected damage in a gene other than the gene regarding the targeted trait. For example, the mutation would deteriorate a lipid-producing fungus in terms of multiplication, spore formation capability, or the like, even though the mutation causes the lipid-producing fungus to produce a different kind of lipid. Therefore, the population creating step adopting the mutagen treating process does not promise that a productive strain can be obtained.

Further, this method creates a population consisted of individuals in which many kinds of mutations are randomly caused. Therefore, if the selecting step after the population creating step did not have appropriate selecting method (screening method) to obtain the mutant (strain) having the targeted character, all the individuals consisting the population should be checked to find out which kind of mutations they have. This is highly labor-consuming.

### (2) Transformation process

Meanwhile, the population creating step adopting the transformation process creates a population of transformant by transfer, into a useful organism to be bred, a DNA fragment necessary to obtain the targeted character (i.e., transformation ). That is, the population creating step adopting the transformation process creates a population controlled to express only specific gene for the targeted character. Therefore, the selecting step after the population creating step is only required to select the desired variety (strain) out of the obtained transformants. Thus, the screening is easy in the selecting step. Further, it is possible to avoid unexpected damage in the gene other than the gene regarding the targeted character. Therefore, this method saves the labor of the breeding significantly.

As described above, the population creating step in the breeding preferably adopts the transformation process. For example, many techniques have been reported as to how to transform filamentous fungi to which *Mortierella* spp. belong.

Specifically, (a) Documents 1 to 3 listed below disclose techniques for transforming filamentous fungi, such as *Aspergillus nidulans, Neurospora crassa,* by particle delivery method. In these techniques, uracil-auxotrophic strains are used as host strains that are to be transformed, and genes complementary to uracil auxotrophy strain are used as marker genes, in order to select the transformant.

Armaleo, D. et al.Curr. Genet., 17, 97-103, 1990 reports the selection of transformants using ura 3 as a marker gene thereby complementing the uracil-auxotrophic characteristic of the hosts : *Saccharomyces cerevisae, Saccharomyces pombe and Neurospara crassa.*

Moreover, (b) Document 4 listed below discloses a technique of the transformation process of *M. alpina* In this technique, plotoplasts are prepared from spores and a gene is transferred into cells by electroporation method. Moreover, screening of the transformants is performed using, as the marker gene, hygromycin B-resistant gene (hpt) derived from *Esherichia coli.* With this, an organism that can grow on a hygromycin containing medium can be selected out as the transformants.
Document 1: Fungaro M. H. et al. Fems microbial Lett., 25, 293-297, 1995
Document 2: Herzog R. W. et al. Appl. Microbiol. Biotechnol., 45, 333-337, 1996
Document 3: Armaleo, D. et al. Curr. Genet., 17, 97-103, 1990
Document 4: Mackenzie D. A. et al. Appl. Environ. Microbiol., 66, 4655-4661, 2000

However, the technique adopting the transformation is disadvantageous that it is difficult to effectively and efficiently perform the breeding of the lipid-producing fungus that produces PUFA.

Specifically, in the technique (a) adopting the transformation of the filamentous fungus by the particle delivery method, the filamentous fungi such as *A. nidulans* and *N. crassa* to be transformed are not suitable for industrial production of the lipids such as PUFA, because lipid productivity of the filamentous fungi is low.

On the other hand, the technique (b) disclosed in Document 4 is a technique for transforming *M. alpina,* which is known as an industrially-applicable lipid-producing fungus. Thus, the technique (b) is advantageous over technique (a) in terms of industrial application.

However, all *Mortierella* spp., typified by *M. alpina,* are not hygromycin sensitive. Therefore, if a hygromycin tolerant fungus of *Mortierella* spp. is used as the host, the hygromycin tolerance cannot be used as the marker.

Most of PUFA are essential fatty acids, which relate to complicated physiological functions in vivo. Therefore, PUFA receives much attention as an important nutrient recently. Therefore, there is a demand for a fermentation technique that produces PUFA more efficiently. To attain such a fermentation technique, it is necessary to establish a technique for efficiently and effectively breeding fungi of *Mortierella* spp., which are highly reliable as a lipid-producing fungus.

The present invention is accomplished in view of the above problem. An object of the present invention is to provide a method of breeding a lipid-producing fungus belonging to *Mortierella* spp., the method being capable of breeding the lipid-producing fungus effectively and efficiently without using an antibiotic.

### DISCLOSURE OF INVENTION

In view of the above problems, the inventors of the present invention found, as a result of diligent works, that it would be possible to efficiently and effectively perform transformation for all strains of the fungi of *Mortierella* spp., if an auxotrophic strain of a fungus of *Mortierella* spp., which is a lipid producing fungus, was obtained and a system was established in which transformation is performed using as a marker a gene complementary to auxotrophy for the auxotrophic strain. The inventors also found that use of this system makes it possible to adopt self-cloning, thereby making the breeding more efficient and effective. The present invention is based on these findings.

A method according to the present invention is a method for breading a lipid-producing fungus belonging to *Mortierella* spp., comprising the step of:
performing transformation, the step of performing the transformation comprising the steps of:
   transferring a marker gene into a host, where the host is an uracil auxotrophic strain of the lipid-producing fungus, and the marker gene is orotidylic acid pyrophosphorylase gene of SEQ ID NO: 5; and
   selecting a transformant by using, as a marker, recovery from the auxotrophy of the host.

The method is preferably arranged such that the lipid-producing fungus is *Mortierella alpina, Mortierella hygrophila,* or *Mortierella chlamydospora.* Moreover, the method is preferably arranged such that the auxotrophic strain is a uracil auxotrophic strain. Furthermore, the method is preferably arranged such that the marker gene is orothidine-5'-phosphate decarboxylase gene or orotidylic acid pyrophosphorylase gene.

Moreover, the method may be arranged such that an electroporation method or particle delivery method is used in the step of transferring.

Furthermore, the method may further includes the step of obtaining 5-FOA tolerant strains from a wild-type lipid-producing fungus.

Additional objects, features, and strengths of the present invention will be made clear by the description below. Further, the advantages of the present invention will be evident from the following explanation in reference to the drawings.

### BEST MODE FOR CARRYING OUT THE INVENTION

One embodiment of the present invention is described below. It should be noted that the embodiment is not to limit the present invention.

In the present invention, the step of performing transformation in which auxotrophy is utilized as a marker is performed as the step of creating a population including a genetic variations to be bred (i.e., as the population creating step). Specifically, an auxotrophic strain of a fungus of *Mortierella* spp. is obtained. This fungus is a lipid-producing fungus. Using this fungus as a host, a gene complementary to the auxotrophy is transferred into the cells of the fungus (transformation). After that, a transformant is selected out of the population using recovery from the auxotrophy in the host as the marker.

The following describes in detail the fungus of *Mortierella* spp., which the present invention uses, the breeding method according to the present invention, and a use thereof.

### (1) Fungus of Mortierella spp.

The fungus of *Mortierella* spp. to be bred by the breeding method of the present invention is not particularly limited. Various filamentous fungi belonging to *Mortierella* spp. may be used as the fungus, for example. *Mortierella* spp. is divided into two subgenus, namely, *Mortierella* and *Micromucor.* All fungi belonging to *Mortierella* subgenus produce fatty acids of carbon number of 20 such as arachidonic acid, meanwhile fungi belonging to Micromucor subgenus produce fatty acids of carbon number of 18 or less. The fungus of *Mortierella* spp. used in the present invention may be any fungi belonging to either *Mortierella* subgenus or *Micromucor* subgenus.

Specific examples of the fungi belonging to *Mortierella* spp. are: *M. alpina, M. elongata, M. exigua, M. hygrophila, M. isabelina, M. turficola, M. gamsii, M. cogitans, M. capitata, M. vinacea, M. chlamydospora,* and the like. It should be noted that the present invention is not limited to these fungi. Among them, *M. alpina* (*Mortierella alpina*), which is widely used as a lipid-producing fungus, can be preferably used in the present invention. It is known that strains of *M. alpina* accumulate, within the fungus bodies, PUFA such as arachidonic acid (ARA). *M. alpina* is widely used not only in the researches of biosynthesis of PUFA but also industrial production of PUFA.

How to obtain the fungus of *Mortierella* spp., including *M. alpina,* is not particularly limited. It is possible to obtain the fungus from depository institutions for microorganisms and the like, such as Institute for fermentation, Osaka, ATCC (American Type Culture Collection), or the like institution. For strains for which patent application have been filed, it is possible to obtain the strain from the International Patent Organism Depository of the National Institute of Advanced Industrial Science and Technology. Further, it is possible to obtain and use an unknown strain belonging to *Mortierella* spp. from a natural environment using a well-known screening method.

### (2) Lipid-Producing Fungus Breeding Method According the Present Invention

The breeding method according to the present invention may be arranged in any way, provided that the population creating step comprises a step of performing transformation (hereinafter, transformation step). The breeding method according to the present invention, however, may more preferably comprise a step of obtaining an auxotrophic strain (hereinafter, auxotrophic strain obtaining step). Of course, the breeding method may comprise a step(s) other than these steps. In the following, these steps are described in detail.

### <Auxotrophic Strain Obtaining Step>

The auxotrophic strain obtaining step is not particularly limited, provided that it obtains an auxotrophic strain (auxotrophic mutant) from a wild-type fungus of *Mortierella* spp. A well-known technique for obtaining an auxotrophic strain may be adopted. Especially, in case where a gene relating to the auxotrophy is known (e.g., in case the gene is found out by the analysis of whole genome, or the like case), it is possible to obtain the auxotrophic strain by knock out the gene.

In the present invention, an auxotrophic strain for uracil (hereinafter, uracil auxotrophic strain) is used, as in Examples later described. The uracil auxotrophic strain is especially advantageous for *Mortierella* spp., because it allows to use 5-fluoro orotic acid (5-FOA) tolerance as a marker for positive selection in the screening.

Therefore, while the auxotrophic strain obtaining step may be arranged to obtain a strain that cannot grow in a synthetic medium in which a particular nutrition is lacked, the auxotrophic strain obtaining step may be arranged to obtain the strain with screening that limits the mutation gene. For example, screening with 5-FOA may be performed in order to obtain a uracil auxotrophic strain in which mutation occurs at URA3 gene or URA 5 gene. Similarly, screening with aminoadipic acid may be performed in order to obtain a lysine auxotrophic strain in which mutation occurs on LYS2 gene or LYS5 gene. It is preferable to obtain an auxotrophic strain in which a mutation gene is limited. This is because it is possible to specify the marker gene for use in the transformation step.

### <Transformation Step 1: Gene transfer Step>

The transformation step is, as described above, the step for performing the transformation in the population creating step for the breeding. The transformation step at least includes a gene transfer step and the selecting-out step. Of course, the transformation step may include a step(s) other than these steps, if necessary.

The gene transfer step in the present invention is not particularly limited, provided that the gene transfer step transfer a marker gene into a host, where the host is an uracile auxotrophic strain of lipid-producing fungus of *Mortierella* spp., and the marker gene orotidic acid pyrophosphorylase gene of SEQ ID NO 5

The marker gene is not particularly limited, as long as the gene is complementary to the mutation (auxotrophic mutation) in the auxotrophic strain. Specifically, examples of the marker gene are: leu2 gene for leucine auxotrophic strain, his3 gene for histidine auxotrophic strain, lys2 gene for lysine auxotrophic strain, trp1 gene for tryptophan auxotrophic strain, ura3 gene or ura5 gene for uracil auxotrophic strain, ade2 gene for adenine auxotrophic strain.

For example, the present invention may be arranged such that the host is the uracil auxotrophic strain and the marker gene is ura5 gene, as described in Examples.

There is no particular limitation as to how to obtain the marker gene. The marker gene may be obtained, by using a well-known method, from a (micro)organism (such as yeast) other than the fungus. A commercially-available marker gene may be used. Alternatively, because self-cloning is applicable to the present invention, the marker gene may be obtained from the fungus of the *Mortierella* spp., which is used as the host. For instance, the later-described Examples are arranged such that ura5 gene was obtained from *M. alpina* (host), using a base sequence of ura5 gene in three kinds of yeasts.

How to transfer the gene (i.e., how to transform) in the gene transfer step is not particularly limited. A conventionally well-known method such as the electroporation method, particle delivery method, or the like method may be used appropriately. For the fungi of *Mortierella* spp., it is preferable that the fungus bodies be protoplasted, if the electroporation method is adopted. A specific method of the particle delivery method is the particle gun method. Of course, the particle delivery method is not limited to the particle gun method.

The marker gene is not particularly limited in terms of its specific structure, provided that the marker gene can be transferred into the fungus bodies of *Mortierella* spp. so that the marker gene can be expressed. In the present invention, the marker gene should be constructed as a gene construction which is prepared by ligating (a) a region (hereinafter, recombinant region) to be recombined with a chromosome with (b) an expression cassette prepared by ligating a promoter thereto. Therefore, the gene construction may be transferred into the host via a vector in which the gene construction has been inserted. Alternatively, the gene construction may be transferred into the host directly.

The expression cassette of the marker gene, which is contained in the gene construction, should be structured such that the promoter is ligated in the upstream of the marker gene. It is preferable that a terminator be ligated in the downstream of the marker gene. Moreover, in addition to the expression cassettes of the marker gene and the recombinant region, the gene construction may contain a multi-closing site, so that a cassette for expression of various gene can be ligated thereto. Further, the gene construction may contain a DNA fragment other than the expression cassettes of the marker gene (which contains the promoter, the marker gene, and the terminator), the recombinant region, and the multi-cloning site. The gene construction is not particularly limited in terms of its specific structure.

There is no particular limitation as to the specific kinds of the DNA fragments mentioned above. For example, there is no particular limitation in case of the promoter, provided that the promoter can cause expression of the marker gene effectively. It is possible to use a known promoter appropriately in the present invention. It should be noted that the present invention is not limited to hisH4.1 promoter used in the later-described Examples. Similarly, there is no particular limitation in case of the terminator, provided that the terminator can function as a transcription termination site. A known terminator may be used in the present invention. It should be noted that present invention is not limited to trpC terminator used in the later-described Examples. Furthermore, there is no particular limitation in the case of the recombinant region, provided that the recombinant region is a DNA segment by which the marker gene to be transferred into the fungus body of the *Mortierella* spp. can be inserted in chromosome. In the later described Example, the recombinant region is 18S ribosomal DNA (18Sr).

There is no particular limitation regarding the recombinant expression vector in case where the gene construction is introduced into a vector to prepare an expression vector. The expression vector may be equivalent to a general recombinant expression vector. The recombinant expression vector may be prepared by using a plasmid, a phage, a cosmid, or the like. However, there is no particular limitation regarding how to prepare the recombinant expression vector. Moreover, the preparation of the recombinant expression vector may be carried out by using a known method. In general, a plasmid can be used preferably in the preparation of the recombinant expression vector.

### <Transformation Step 2: Selecting-out step>

The selecting-out step in the transformation step is not particularly limited, provided that the selecting-out step selects out the transformant using the recovery from the auxotrophy of the host as the marker. The selecting-out step may be carried out by using a known method appropriately. Specifically, the selecting-out step may be carried out by, in a synthetic medium, the transformants obtained after the gene transfer step, the synthetic medium being such that a specific nutrition is omitted therein in view of the auxotrophy that is to be used as the marker. With this, it is possible to screen the transformants easily and efficiently.

As described above, the auxotrophy is used as the marker in the transformation step. The auxotrophic marker is advantageous in easy operation and low background. Further, auxotrophic marker is advantageous in that a gene derived from the fungus of *Mortierella* spp. used as the host can be utilized. Moreover, the use of the auxotrophy as the marker is preferable for food product development, compared with the case where the antibody tolerance is used as the marker.

This is more specifically explained here referring to the case of the technique of Document 4 explained in Background Art, that is, the case where the hygromycin tolerance is used as the marker, by way of example. In order to stably grow the transformants to be selected, it is necessary to add the hygromycin in the medium. As a result, the lipid produced by using the transformants would be contaminated with hygromycin. For the lipid to be consumed as food, it is better to avoid that the lipid is contaminated with hygromycin.

For example, in case of the auxotrophy is the uracil auxotrophic strain, a uracil-lacking medium is used in the actual production. This makes it possible to produce the lipid while selectively growing the fungus in which the gene (such as ura5 gene or the like) complementary to the auxotrophy is transferred. That is, by using the auxotrophy as the marker, it is possible to avoid hygromycin contamination and perform selective culturing always. Thus, it is preferable to use the auxotrophic marker as in the present invention.

### <Selecting Step>

According to the breeding method of the present invention for breeding the lipid-producing fungus, populations of strains of various characters can be obtained by transferring various genes in the transformation step in which the auxotrophy is used as the marker. Therefore, the present invention may be arranged to comprise the selecting-out step for selecting, from the obtained population, a variety having a desired character. The "selecting step" is different from the "selecting-out step" in the transformation step. The selecting step is not for selecting out the transformant, using the auxotrophy as the marker. The selecting step is for selecting a transformant having more preferable character, out of the plurality of the obtained transformants.

There is no particular limitation regarding how to carry out the selecting step specifically. In order to select out the transformant having the preferable character, the selecting step may be arranged, according to the purpose of the breeding, to be carried out by a known method under conditions that allow selection of the transformant having the preferable character.

### (3) Use of Present Invention

The breeding method according to the present invention for breeding a lipid-producing fungus is, as described above, arranged such that a desired variety having a desirable character is selected (screened) out of a population having a genetic variations. As the marker in the selection (screening), auxotophy is used. With this arrangement, it is possible to efficiently and effectively produce a novel variety (novel strain) obtained from, as the wild type, a lipid-producing fungus belonging to *Mortierella* spp. *Mortierella* spp. are well known as a lipid producing fungus. Especially, *M. alpina.* is known for its high reliability as a lipid producing fungus. It is possible to produce efficiently and easily a strain improved, for example, in the lipid productivity.

Moreover, the breeding method according to the present invention can perform the transformation using only a gene of the lipid-producing fungus to be transformed. Moreover, self-cloning is applicable in the present invention. Thus, it is possible to obtain an appropriate DNA fragment from the wild-type fungus of *Mortierelld* spp., and transfer the DNA fragment into the auxotrophic strain, thereby controlling expression of a specific gene (high expression, controlling of expression timing, inhibition of expression, etc.). This makes it possible to obtain a transformant, that is, a novel strain, which can attain such improvements such as an increase in lipid production, modification of the lipid production in terms of kind or composition of the lipid to produce, etc. Of course, the present invention may be arranged such that a DNA fragment derived from other variety is transferred into the auxotrophic strain, without self-cloning.

The breeding method according to the present invention is not particularly limited in terms of technique to adopt and may adopt a known technique. For example, in the case of self-cloning, how to obtain an appropriate DNA fragment from the wild-type fungus of *Mortierella* spp. (host) is not particularly limited and may be performed by a known technique. Moreover, the appropriate DNA fragment (for example a gene to be transferred, etc.) is not particularly limited, provided that the DNA fragment can realize a targeted and desired character, or has a possibility to realized the character.

For example, the DNA fragment may be such that an expression cassette ligated at least with a promoter and preferably with a terminator is inserted in a recombinant expression vector. However, the present invention is not limited to this. Further, the present invention is not limited to GLELO gene, which was used as the DNA fragment in the later-described Examples.

The present invention is described in detail referring to Examples. It should be noted that these Examples are not to limit the present invention.

### [Example 1]

Here, a specific example of the breeding method of the present invention is explained. In the present example, a uracil auxotrophic strain was obtained, and transformation and selection of the uracil auxotrophic strain were performed.

### (1) Obtaining Uracil Auxotrophic strain (Auxotrophic Strain Obtaining Step)

In order to form spores of *M. alpina, M. alpina* was inoculated on Czapek-Dox medium (3% sucrose, 0.2% NaNO₃, 0.1%KH₂PO₄, 0.05%KCl, 0.05%MgSO₄·7H₂O, 0.001%FeSO₄·7H₂O, 2% agar; adjusted to pH6.0) and incubated at 28°C for approximately 2 weeks. This was then suspended in Tween 80 aqueous solution (1drop/100ml H₂O). Then, hyphae were removed using a glass filter (Iwaki Glass Co., Ltd.; Product No.: 3G1), thereby to obtain a spore solution. Mutagen treatment was carried out with N-methyl-N'-nitro-N-nitrosoguanidine (MNNG) with respect to spores of 10 × 10⁸ to 109 according to the method of Jareonkitmongkol et at. (S. Jareonkitmongkol et al. JAOCS, 69, 939-944, 1992).

The spores treated by mutagen were spread on GY medium (2% glucose, 1% yeast extract, 2% agar; adjusted to pH 6.0) containing 5-FOA of 1.0mg/ml and uracil of 0.05mg/ml, and incubated at 28°C for 4 days. Thereby, six 5-FOA tolerant strains were obtained. The obtained strains were evaluated with respect to uracil auxotrophy.

That is, growths of these strains on SC medium and uracil-added SC medium were compared. (SC medium: Yeast Nitrogen Base w/o Amino Acids and Ammonium Sulfate (Difco) 5.0g, (NH₄)₂SO₄ 1.7g, glucose 20g, agar 20g, adenine 20mg, tyrosine 30mg, methionine 1.0mg, arginine 2.0mg, histidine 2.0mg, lysine 4.0mg, tryptophan 4.0mg, threonine 5.0mg, isoleucine 6.0mg, leucine 6.0mg, phenylalanine 6.0mg/liter; uracil-added SC medium: uracil 50mg/liter).

The evaluation showed that all the six 5-FOA tolerant strains could grow on the uracil-added media like the parent strain, but grew poorly on the medium containing no uracil. Two of the six 5-FOA tolerant strains could not grow on the medium containing no uracil. These strains were named as Δura-1 strain and Δura-2 strain.

### (2) Evaluation of Enzyme Activity

When a strain shows 5-FOA tolerance and uracil auxotrophy as its phenotypes, one possibility is that the strain lacks an activity of orothidine-5'-phosphate decarboxylase (which is an enzyme coded by ura3 gene; abbreviated to ura3p hereinafter) or orotidylic acid pyrophosphorylase (which is an enzyme coded by ura5 gene; abbreviated to ura5p). Thus, the activities of these enzymes in the Δura-1 strain and Δura-2 strain were evaluated.

These strains were incubated on uracil-added GY medium at 28°C for 4 days with shaking. Preparation of cell-free extract solution of the fungus bodies was carried out according to a method modified from the method of Wynn et al. (J. P. Wynn et al. Microbiology, 146, 2325-2331, 2000).

Specifically, the fungus bodies obtained from the 4-day incubation at 28°C on the GY liquid medium containing uracil of 0.05mg/ ml were suspended in 0.1M trisbuffer (pH 7.5) containing β-mercaptoethanol. Then, the cells were crushed by applying 35MPa using a French press. The obtained solution was centrifuged to obtain a supernatant, which was the cell-free extract solution. Further, the supernatant was subjected to super centrifugation under conditions 100,000×g for 60 min, thereby to remove organella. In this way, a soluble fraction was obtained.

The ura3p activity was measured using the obtained soluble fraction according to the method of Yoshimoto et. el. (A. Yoshimoto et al. Methods in Enzymology Vol 51, 74-79, ACADEMIC PRESS 1978). The ura5p activity was measured using the obtained soluble fraction according to the method of Umezu et. al. (K. Umezu et al. J. Biochem., 70, 249-262, 1971). The results, that is, comparison of the enzymic activities regarding pyrimidine biosynthesis, are given in Table 1. As seen from the results in Table 1, both the two strains had the ura3p activity equivalently to the parent strain, but no ura5p activity was detected in these two strains.

Further, 10⁸ to 10⁹ spores prepared separately were subjected to the same mutagen treatment. This gave five 5-FOA tolerant strains, three of which could not grow on the medium containing no uracil.

**Table 1**

| Strain | ura3p (mU/mg) | ura5p (mU/mg) |
|---|---|---|
| wild type | 15.2 | 20.2 |
| Δura-1 | 12.6 | - |
| Δura-2 | 21.2 | - |

| | | |
|---|---|---|
| 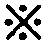 - : not detected | | |

### (3) Obtaining genome DNA of M. alpina and cDNA library

*M. alpina* and the uracil auxotrophic strains (the Δura-1 strain and Δura-2 strain) derived from *M. alpina* as the wild type were respectively incubated in GY liquid media at 28°C for 5 days. From the obtained fungus bodies, genome DNAs were prepared according to the method of Sakuradani et. al. (E. Sakuradani et al. Eur. J. Biochem., 260, 208-216, 1999).

Spores of *M. alpina* were inoculated in a liquid medium (2% glucose, 1% yeast extract; adjusted to pH 6.0) and incubated at 28°C for 4 days, and fungus bodies were collected therefrom. Then, all RNAs were prepared by hydrochloride guanidine/CsCl method. Using Oligotex-dT30<Super>mRNA Purification Kit (From Total RNA) (Product Name (Takara Bio Inc.)), mRNAs were purified from total RNA. Then, a cDNA library was prepared using ZAP-cDNA Synhesis Kit (Product Name (STRATAGENE)).

### (4) Obtaining ura5 cDNA from M. alpina

In amino acid sequences of ura5p of three kinds of yeasts, namely, *Glomerella graminicola, Saccharomyces cerevisiae,* and *Sordaria macrospora,* the followings are amino acid sequences of two regions having a high homology with each other.
Region 1: FGPAYKGIP (see SEQ. ID. NO. 1)
Region 2: KEAKDHGEG (see SEQ. ID NO. 2)
Based on these amino acid sequences of the two regions, sense primers and antisense primers respectively having the following base sequences were synthesized.
Senseprimer: 5' -TTYGGHCCIGCITAYAARGGHATYCC-3' (see SEQ. ID. NO. 3)
Antisenseprimer: 5' -CCCTCDCCRTGRTCYTTIGCYTCYTT-3' (see SEQ. ID. NO. 4)
"I" in the primer stands for inosine. Note that in the Sequence Table, "n" stands for the site corresponds to inosine.

Then, PCR was carried out with (a) the genome DNA of *M. alpina* as a template, (b) the two primers mentioned above, and (c) Ex Taq polymerase (Takara Bio Inc.), using T Gradient thermocycler (product name; Biometra). The PCR was carried out with 35 cycles of 1 min at 94°C, 1 min at 52°C, and 2 min at 72°C, and then 10 minute elongation at 72°C thereafter.

This produced approximately 130bp DNA fragment, a base sequence of which was then determined. The DNA fragment was found to be highly homologous with the known ura5 gene. Using the DNA as a probe, the cDNA library of *M. alpina* prepared in (3) was screened by plaque hybridization, thereby obtaining plasmid pBMAURA5 including cDNA of the ura5 gene.

cDNA of the ura5 gene had the base sequence shown in SEQ. ID. NO. 5. The ura5 gene codes ura5p, which has the amino acid sequence shown in SEQ. ID. NO. 6.

### (5) Cloning of ura5 genome DNA

Based on the cDNA sequence (SEQ. ID. NO. 5) of the ura5 gene, the following primer CRON1 and CRON2 were synthesized:
Primer CRON 1: CTTCTCTTCAGCCCCCAGCCGCATCGGTCC (SEQ. ID. NO. 7)
Primer CRON 2: GAGTCCACGAAAACTGCTGATGAGCAGCAC (SEQ. ID. NO. 8)

Using the genome DNA of *M. alpina* as a template, ura5 genome DNA was cloned by PCR. The PCR was carried out with 30 cycles of 1 min at 94°C, 1 min at 55°C, and 2 min at 72°C, and then 10 minute elongation at 72°C thereafter. As a result, it was found that no intron existed in ura5 genome gene.

### (6) Identification of Mutation Site in Auxotrophic strain

Using the primers CRON 1 and CRON 2, PCR was performed using, as templates, the genome DNA of Δura-1 strain and Δura-2 strain, which were uracil auxotrophic strains. Thereby, ura5 genome genes from the respective strains were cloned. The PCR was carried out with 30 cycles 1 min at 94°C, 1 min at 55°C, and 2 min at 72°C, and then 10 minute elongation at 72°C thereafter. It was found that in the Δura-1 strain one adenine was inserted at 93th from the initiation codon, and a frame shift was observed in ORF. On the other hand, in the Δura-2 strain, guanine at 398th was replaced with adenine, and glycine at 133th in a motif sequence was replaced with aspartic acid. This suggested that these mutations inactivated the ura5p activity in these two strains.

### (7) Recombinant Expression Vector: Constructing pDura5

Plasmid pD4 (supplied from D. B. Archer, see Document 4) was digested with restriction enzymes NcoI and BamHI, and its ends were blunted by using a DNA Blunting Kit (Product Name; Takara Bio Inc.). Then, a DNA fragment of 5.4 kbp except hpt mod fragment, which is the hygromycin B tolerant gene was purified by using GFX PCR DNA and Gel Band Purification Kit (Product Name (made by Amasham Pharmacia Biotech & Science). With the restriction enzymes EcoRI and XhoI, pBMAURA5 obtained in (4) was digested. After blunting its ends, a fragment of approximately 0.65 kbp including ura5 cDNA was purified. These two fragments were ligated with each other by using ligation high (Product Name (Toyobo Ltd.). The direction of ura5 gene is confirmed to select, as plasmid pDura5, a fragment that contains hisH4.1 promoter on the 5' side of ura5 gene, and trpC terminator on the 3' side of ura5 gene.

### (8) Transformation using pDura 5 (Transformation Step)

SC agar medium was used as the selective medium for the transformant. Spores (× 10⁸) of Δura-1 strain were spread on the SC agar medium. pDura5 was transferred into the host by the particle deliver method (transformation). The gene transfer, carried out by using PDS-1000/He particle delivery system (Product Name; BIO RAD), was performed under the following conditions: Helium pressure: 7590kPa (1100 psi); vacuum in chamber: 28inch HG; Distance from target: 3cm; and tungsten particle diameter 1.1µm. Four colonies grew on the SC agar medium after 2-3 day incubation at 28°C after the gene transfer. These colonies were collected as the transformant, which were labeled as #1 to #4.

The transformant # 1 to #4 were examined by using PCR as to whether the gene was transferred into them. That is, a genome DNA was prepared from each transformant. PCR was carried out using the genome DNA as the template, EX Taq polymerase (Takara Bio Inc.), and the following primers RDNA1 and RDNA2.
Primer RDNA1: ACAGGTACACTTGTTTAGAG (SEQ. ID. NO. 9)
Primer RDNA2: CGCTGCGTTCTTCATCGATG (SEQ. ID. NO. 10)
The PCR was carried out with 35 cycles of 1 min at 94°C, 1 min at 54°C, and 2min at 72°C, and then 10 minute elongation at 72°C thereafter. As a control, PCR was performed on the Δura-1 strain.

It was found that no DNA amplification was observed for the Δura-1 strain used as the host. Meanwhile, DNA fragment of 1.5kbp was amplified in each of the transformant #1 to #4. This confirmed that recombination with plasmid pDura5 occurred in 18SrDNA region of each of the transformant #1 to #4, thereby transferring the ura5 gene.

Further, the transformant # 1 to #4 were incubated in SC liquid media at 28°C for 4 days with shaking, in order to evaluate the ura5p enzyme activity. The results, that is, the comparison of the ura5p enzyme activities is shown in Table 2. As seen from the result in the Table 2, the transformant #1 to #4 had recovered the activity equivalently to or several times greater than the wild type. This proved that the transferred ura5 gene functioned effectively.

**Table 2**

| Strain | ura5p |
|---|---|
| wild type | 59.0 |
| Transformation Strain #1 | 556.0 |
| Transformation Strain #2 | 45.6 |
| Transformation Strain #3 | 55.2 |
| Transformation Strain #4 | 295.0 |

Further, the same gene transfer operation for approximately 10⁸ spores of Δura-1 strain was repeated two times. The resultants were respectively incubated for 2-3 day at 28°C, which grew 3 and 4 colonies respectively. This confirmed that the transformation is reproducible.

### [Example 2]

The present example describes an example where a novel strain, namely GLELO gene-transferred strain was prepared by the breeding method in which the uracil auxotrophic strain prepared in Example 1 was used.

### (1) Constructing pDura 5GLELO vector

GLELO gene codes for a fatty acid chain elongation enzyme, which converts γ-linolenic acid into dihomo-γ-linolenic acid. GLELO gene was prepared by PCR amplification using cDNA of *M. alpina* as a template based on the base sequence of the sequence GB (ID: AF206662) disclosed in J. M. Parker-Barnes et al. Proc. Natl. Acad. Sci. USA., 97 (15), 8284-8289, 2000. In the PCR amplification, the following primers MAGLELO1 and MAGLELO2 were used.
Primer MAGLELO1: CCATGGATGGAGTCGATTGC-GCCATTCC (SEQ. ID. NO. 11)
Primer MAGLELO2: GGATCCTTACTGCAACTTCCTTGCCTTCTC (SEQ. ID. NO. 12)

The amplified GLELO gene was digested with restriction enzymes NcoI and BamHI, thereby to obtain a fragment of approximately 1kb. Moreover, pD4 was digested with the restriction enzymes NcoI and BamHI, thereby to obtain a fragment of approximately 7.7kb. Each fragment was ligated by using ligation high (Product Name; Toyobo Ltd.), thereby to obtain plasmid pDGLELO.

On the other hand, pDura5 was partially digested with EcoRI. Then, DNA fragments of approximately 6.2 kb, which were cut only at one of two EcoRI sites, were purified. After ends of the fragments were blunted by using DNA Blunting Kit (Product Name; Takara Bio Inc.), the fragments were subjected to self ligation by using ligation high (Product Name; Toyobo Ltd.). Then, fragments in which only that one of the sites which was located in the 5' side of histone 4.1 promoter were collected as pDura 5'. Moreover, pDGLELO was digested with EcoRI, thereby to obtain fragments of 2. 7kb. The fragments were then inserted at EcoRI site of pDura5'. From these fragments, a plasmid in which GLELO expression cassettes and ura5 expression cassettes were aligned in the same direction was selected as recombinant expression vector pDura5GLELO.

### (2) Transformation using pDura5GLELO (Transformation Step)

Following the same method as in (8) in Example 1, transformation was carried out for the Δura-1 strain thereby obtaining three transformant. For the sake of easy explanation, these transformants were labeled as #5 to #7, respectively.

The transformant #5 to #7 were examined by using PCR as to whether or not pDura5GLELO was transferred into them. The PCR was carried out in the same manner as in (8) in Example 1, using the primers RDNA1 and RDNA2. It was found that the DNA fragment of 1.5kbp was amplified in each of the three strains #5 to #7. This confirmed that recombination with pDura5GLELO occurred in 18SrDNA region of the three strains #5 to #7, thereby transferring the ura5 gene.

### (3) Analysis of Expression of GLELO gene

Using RNeasy plant mini kit (Product Name (made by QIAGEN)), total RNA was extracted from each of the two ura5 gene-transferred strains #1 and #2 created in Example 1 and the GLELO gene-transferred strains #5 to #7 created in (2) above. The strains #5 to #7 had been incubated under the same incubation conditions as in the lipid analysis.

Using Super Script First-Strand Synthesis System for RT-PCR (Product Name (made by Invitrogen)), reverse transcription of the total RNA (1µg) was performed with random hexamer, thereby synthesizing cDNA. Then, PCR was performed using 1µl (1/20 in amount) of synthesized cDNA as a template, EX Taq polymerase (Takara Bio Inc.), and the following primers MAGLELO 5 and MAGLELO 6:
Primer MAGLELO 5: CTTTGTGGGCATGCAGATCA (SEQ. ID. No. 13)
Primer MAGLELO 6: TGAAGATGGAGCTGTGGTGGTA (SEQ. ID. NO. 14)
The PCR was carried out with 20 cycles of 1min at 94°C, 1min at 55°C, 2min at 72°C, and then 10 min elongation at 72°C thereafter.

Electrophoresis of the product of the PCR was carried out. Fluorescent density for a band of fragment of approximately 300bp was compared. As a result, the band was very light in the fluorescent density in both the ura5 gene-transferred strains, while the band was in a clear density in all the three GLELO gene-transferred strains. On the other hand, when the PCR was carried out in the same manner but with 30 cycles, the band was in clear density in all the strains. This suggests that expression amount of GLELO gene was increased in the GLELO gene-transferred strains.

### (4) Lipid Analysis of GLELO Gene Transfer-Strain

The spores of the two ura5 gene-transferred strains #1 and #2 created in Example 1, and the GLELO gene-transferred strains #5 to #7 obtained in (2) above were respectively inoculated in liquid media (5% glucose, 1% yeast extract; adjusted to pH 6.0), and incubated at 28°C for 7 days with shaking. After the incubation, the fungus bodies were collected by filtration. The collected fungus bodies were then dried and weighted. According to hydrochloric acid-methanol method, fatty acid residues in the fungus bodies were converted into methyl ester. The obtained methyl ester was extracted with hexane, which was distilled off thereafter, thereby obtaining methyl ester fatty acids. The obtained methyl ester fatty acids were analyzed with gas chromatography. The result of the analysis is shown in Table 3, showing weight of dried fungus bodies when the respective gene-transferred strains (transformant) were grown, and fatty acid compositions and total lipid production amount in the fungus bodies of each strain.

In Table 3, 16:0 is palmitic acid, 18:0 is stearic acid, 18:1 is oleic acid, 18:2 is linoleic acid, 18:3 is γ-linoleic acid, 20:3 is dihomo-γ-linoleic acid, 20:4 is arachidonic acid, and 24:0 is lignoceric acid.

**Table 3**

| Strain | | ura 5 Gene Transferred Strain | | GLELO gene Transferred Strain | | |
|---|---|---|---|---|---|---|
| | | #1 | #2 | #5 | #6 | #7 |
| Weight of Dried Fungus | | 14.8 | 11.7 | 18.1 | 18.6 | 16.8 |
| body | | | | | | |
| (mg/Medium 1mL) | | | | | | |
| Fatty Acid Composition | | | | | | |
| (%) | | 23.0 | 21.1 | 23.1 | 22.3 | 22.8 |
| | 16 : 0 | 5.7 | 5.9 | 6.5 | 6.1 | 8.1 |
| | 18 : 0 | 34.0 | 33.8 | 32.8 | 32.4 | 30.0 |
| | 18 : 1 | 6.5 | 8.0 | 4.5 | 5.8 | 5.1 |
| | 18 : 2 | 3.8 | 4.1 | 1.9 | 2.7 | 2.4 |
| | 18 : 3 | 3.8 | 3.6 | 3.8 | 4.1 | 4.3 |
| | 20 : 3 | 16.3 | 14.5 | 19.3 | 20.3 | 20.7 |
| | 20 : 4 | 2.6 | 2.6 | 3.7 | 3.0 | 3.0 |
| | 24 : 0 | 4.3 | 6.4 | 4.4 | 3.3 | 3.6 |
| | Other | | | | | |
| Lipid Production Amount | | | | | | |
| (mg/Medium1mL) | | | | | | |
| | Total Lipid | 5.07 | 3.48 | 7.32 | 7.94 | 6.33 |
| | 20 : 3 | 0.20 | 0.13 | 0.28 | 0.33 | 0.27 |
| | 20 : 4 | 0.82 | 0.50 | 1.41 | 1.61 | 1.31 |
| Lipid Content | | | | | | |
| (mg/Dried Fungus Body g) | | | | | | |
| | Total Lipid | 342 | 297 | 406 | 427 | 377 |
| | 20 : 3 | 13.2 | 10.6 | 15.5 | 17.9 | 16.1 |
| | 20 : 4 | 55.6 | 42.9 | 78.1 | 86.7 | 78.0 |
| 20 : 3/18 : 3 Ratio | | 1.01 | 0.88 | 1.99 | 1.57 | 1.77 |
| (20 : 3+20 : 4) /18 : 3 Ratio | | 5.3 | 4.4 | 12.0 | 9.2 | 10.3 |

As seen from Table 3, the transfer of GLELO gene increased the weight of dried fungus bodies, and the ratios of dihomo-γ-linoleic acid and arachidonic acid in the total fatty acids. However, the transfer of GLELO gene decreased the ratio of γ-linoleic acid in the total fatty acids. Furthermore, total amount of lipid produced in the produced and total lipid content were increased as a result of the transfer of GLELO gene. Moreover, the transfer of GLELO gene increased the production amount and contents of dihomo-γ-linoleic acid and arachidonic acid. The GLELO gene-transferred strains showed a significant increase in the ratio (20:3/18:3) between dihomo-γ-linoleic acid and γ-linoleic acid, which are the product of the chain-length elongation reaction and a ground substance thereof, or the ratio ((20:3+20:4)/18:3) of a sum of dihomo-γ-linoleic acid and arachidonic acid, which is a metabolite from dihomo-γ-linoleic acid, over γ-linoleic acid.

### [Example 3]

The present example explains a specific example in which the breeding method according to the present invention was applied to a fungus of *Mortierella* spp. other than *M. alpina.*

### (1) Obtaining Uracil Auxotrophic strain (Auxotrophic Strain Obtaining Step)

In order to form spores of M. *hygrophila* and *M. chlamydospora,* these fungi were respectively inoculated on Czapek-Dox media (3% sucrose, 0.2% NaNO₃, 0.1%KH₂PO₄, 0.05%KCl, 0.05%MgSO₄·7H₂O, 0.001%FeSO₄·7H₂O, 2% agar; adjusted to pH6.0) and incubated at 28°C for approximately 2 weeks. These were then suspended in Tween 80 aqueous solution (1drop/100ml H₂O). Then, hyphae were removed using a glass filter (Iwaki Glass Co., Ltd.; Product No.: 3G1), thereby to obtain a spore solution. Mutagen treatment was carried out with N-methyl-N'-nitro-N-nitrosoguanidine (MNNG) with respect to spores of 10 × 10⁸ to 10⁹ according to the method of Jareonkitmongkol et at. (S. Jareonkitmongkol et al. JAOCS, 69, 939-944, 1992).

The spores treated by mutagen were spread on GY media (2% glucose, 1% yeast extract, 2% agar; adjusted to pH 6.0) containing 5-FOA of 1.0mg/ml and uracil of 0.05mg/ml, and incubated at 28°C for 4 days. Thereby, two 5-FOA tolerant strains were obtained from M. *hygrophila* and one 5-FOA tolerant strain was obtained from *M. chlamydospora.* The obtained strains were evaluated with respect to uracil auxotrophy.

That is, growths of these strains on SC medium and uracil-added SC medium were compared (SC medium: Yeast Nitrogen Base w/o Amino Acids and Ammonium Sulfate (Difco) 5.0g, (NH₄)₂SO₄ 1.7g, glucose 20g, agar 20g, adenine 20mg, tyrosine 30mg, methionine 1.0mg, arginine 2.0mg, histidine 2.0mg, lysine 4.0mg, tryptophan 4.0mg, threonine 5.0mg, isoleucine 6.0mg, leucine 6.0mg, phenylalanine 6.0mg/liter; uracil-added SC medium: uracil 50mg/liter).

The evaluation showed that all the obtained 5-FOA tolerant strains could grow on the uracil-added medium like the parent strain, but grew poorly on the medium containing no uracil. One of the two strains from M. *hygrophila* and the strain from *M. chlamydospora* could not grow on the medium containing no uracil. This one of the two strains from M. *hygrophila* was named as MH-Δura-1 strain, while the strain from *M. chlamydospora* was named as MC-Δura-1 strain.

### (2) Evaluation of Enzyme Activity

These two strains were incubated on uracil-added GY medium at 28°C for 4 days with shaking. Preparation of cell-free extract solution of the fungus bodies was carried out according to a method modified from the method of Wynn et al. (J. P. Wynn et al. Microbiology, 146, 2325-2331, 2000).

Specifically, the fungus bodies obtained from the 4-day incubation at 28°C on the GY liquid medium containing uracil of 0.05mg/ml were suspended in 0.1M trisbuffer (pH 7.5) containing β-mercaptoethanol. Then, the cells were crushed by applying 35MPa using a French press. The obtained solution was centrifuged to obtain a supernatant, which was the cell-free extract solution. Further, super centrifugation was carried out under conditions of 100,000×g for 60 min, thereby to remove organella. In this way, a soluble fraction was obtained.

Using the obtained soluble fraction, orotidylic acid pyrophosphorylase (ura5p) activity was evaluated according to the method of Umezu et al. (K. Umezu et al. J. Biochem., 70, 249-262, 1971). The evaluation showed that the ura5p activity was below measurable limit in each of these strains.

### (3) Transformation using pDura 5 (Transformation Step)

M. *hygrophila* MH-Δura-1 or *M. chlamydospora* MC-Δura-1 was inoculated on Czapek-Dox medium and incubated at 28°C for 2 weeks, thereby forming spores. The spores were collected as described in (1) above.

SC agar medium was used as the selective medium for the transformed strains. pDura5 was transferred into the host by the particle delivery method (transformation).The gene transfer, carried out by using PDS-1000/He particle delivery system (Product Name; BIO RAD), was performed under the following conditions: Helium pressure: 7590kPa (1100 psi); vacuum in chamber: 28inch HG; Distance from target: 3cm; and tungsten particle diameter 1.1µm.) After the gene transfer, about dozen colonies grew on the SC agar medium after 2-3 day incubation at 28°C for each strain. These colonies were collected as the transformant.

The embodiments and concrete examples of implementation discussed in the foregoing detailed explanation serve solely to illustrate the technical details of the present invention, which should not be narrowly interpreted within the limits of such embodiments and concrete examples, but rather may be applied in many variations within the spirit of the present invention, provided such variations do not exceed the scope of the patent claims set forth below.

### INDUSTRIAL APPLICABILITY

A method according to the present invention is a method for breading a lipid-producing fungus belonging to *Mortierella* spp., comprising the step of performing transformation, the step of performing the transformation comprising the steps of transferring a marker gene into a host, where the host is an uracil auxotrophic strain of the lipid-producing fungus, and the marker gene is orotidylic acid pyrophosphorylase gene of SEQ ID NO: 5 (gene transfer step); and selecting a transformant by using, as a marker, recovery from the auxotrophy of the host (selecting out step).

With this arrangement, the present invention makes it possible to efficiently and effectively produce a novel variety (novel strain) from the lipid-producing fungus belonging to *Mortierella* spp. as the wild type.

Moreover, self-cloning is applicable in the present invention. Thus, it is possible to obtain an appropriate DNA fragment from the wild-type fungus of *Mortierella* spp., and transfer the DNA fragment into the auxotrophic strain, thereby controlling expression of a specific gene (high expression, controlling of expression timing, inhibition of expression, etc.). This makes it possible to obtain a transformant, that is, a novel strain, which can attain such improvements such as an increase in lipid production, modification of the lipid production in terms of kind or composition of the lipid to produce, etc.

*Mortierella* spp., are well known as a lipid producing fungus, and includes highly-reliable lipid producing fungus such as *M. alpina.* Thus, according to the present invention, for example, it is possible to attain effective and easy production of a strain which is improved in the lipid productivity, or like effect.

Therefore, the present invention is applicable to industries relating to various fermentation techniques using *Mortierella* spp., and food industry, pharmaceutical industry, and the like industries using the fermentation techniques.

## Claims

1. A method for breeding a lipid-producing fungus belonging to *Mortierella* spp., comprising the step of:
performing transformation, the step of performing the transformation comprising the steps of:
- transferring a marker gene into a host, where the host is an uracil auxotrophic strain of the lipid-producing fungus, and the marker gene is orotidylic acid pyrophosphorylase gene of SEO ID NO: 5; and
- selecting a transformant by using, as a marker, recovery from the auxotrophy of the host.

2. The method as set forth in Claim 1, wherein:
the lipid-producing fungus is *Mortierella alpina, Mortierella hygrophila,* or *Mortierella chlamydospora.*

3. The method as set forth in Claim 1 or 2, wherein:
an electroporation method or particle delivery method is used in the step of transferring.

4. The method as set forth in any one of Claims 1 to 3, further comprising the step of:
obtaining 5-FOA tolerant strains from a wild-type lipid-producing fungus

## Patentansprüche

1. Verfahren zum Züchten eines lipidproduzierenden Pilzes, der zu den *Mortierellae* Spezies gehört, mit dem Schritt:
Durchführen einer Transformation, wobei der Schritt des Durchführens der Transformation die Schritte umfasst:
- Transferieren eines Markergens in einen Wirt, wobei der Wirt eine uracilauxotrophe Kette des lipidproduzierenden Pilzes ist und das Markergen ein Orotidylsäure-Pyrophosphorylasegen der SEQ ID NO: 5 ist; und
- Auswählen eines Transformanten durch Verwenden einer Ausbeute von der Auxotrohpie des Wirtes als Marker.

2. Verfahren nach Anspruch 1, in welchen:
Der lipidproduzierende Pilz *Mortierella Alpina, Mortierella Hygrophila,* oder *Mortierella Chlamydospora* ist.

3. Verfahren nach Anspruch 1 oder 2, in welchem ein Elektroporationsverfahren oder ein Teilchen Abgabeverfahren im Schritt des Transferierens verwendet wird.

4. Verfahren nach einem der Ansprüche 1 bis 3, ferner mit dem Schritt:
Erhalten von 5-FOA toleranten Ketten des lipidproduzierenden Pilzes des Wildtyps.

## Revendications

1. Procédé de culture d'un champignon producteur de lipides appartenant à *Mortierella spp.,* comprenant l'étape qui consiste:
à réaliser une transformation, l'étape de réalisation d'une transformation comprenant les étapes qui consistent :
à transférer un gène marqueur dans un hôte, où l'hôte est une souche auxotrophe à l'uracile du champignon producteur de lipides, et le gène marqueur est un gène pyrophosphorylase d'acide orotidylique de SEQ ID NO:5 ; et
à choisir un transformé en utilisant comme marqueur, la récupération de l'auxotrophie de l'hôte.

2. Procédé selon la revendication 1, dans lequel :
le champignon producteur de lipides est *Mortierella alpina, Mortierella hygrophila,* ou *Mortierella chlamydospora.*

3. Procédé selon la revendication 1 ou 2, dans lequel:
un procédé d'électroporation ou un procédé de distribution de particules est utilisé dans l'étape de transfert.

4. Procédé selon l'une quelconque des revendications 1 à 3, comprenant en outre l'étape qui consiste :
à obtenir des souches tolérantes à 5-FOA à partir d'un champignon producteur de lipides de type sauvage.


### SEQUENCE LISTING

<110> SUNTORY LIMITED
<120> Breeding method of Lipid product mold
<130> SU0410
<150> JP 2003-299345
   <151> 2003-08-22
<160> 14
<170> PatentIn Ver. 2.1
<210> 1
   <211> 9
   <212> PRT
   <213> Artificial Sequence
<220>
   <223> Description of Artificial Sequence: Motif for Primer Sequence
<400> 1
   Phe Gly Pro Ala Tyr Lys Gly Ile Pro
   1 5
<210> 2
   <211> 9
   <212> PRT
<213> Artificial Sequence
<220>
   <223> Description of Artificial Sequence: Motif for Primer Sequence
<400> 2
   Lys Glu Ala Lys Asp His Gly Glu Gly
   1 5
<210> 3
   <211> 26
   <212> DNA
   <213> Artificial Sequence
<220>
   <221> n
   <222> 9
<220>
   <221> n
   <222> 12
<220>
   <223> Description of Artificial Sequence : Artificially Synthesized Primer Sequence
<400> 3
   ttygghccng cntayaargg hatycc 26
<210> 4
   <211> 26
   <212> DNA
   <213> Artificial Sequence
<220>
   <221> n
   <222> 18
<220>
   <223> Description of Artificial Sequence : Artificially Synthesized Primer Sequence
<400> 4
   ccctcdccrt grtcyttngc ytcytt 26
<210> 5
   <211> 925
   <212> DNA
   <213> Mortierella alpina
<400> 5
   acaaccttct cctcagcccc cagccgcatc ggtcccaccg ccgcaaccca tcagcacaca 60
   atggccatca aggattacca gcgcgagttc attgagtttg ccatcaagaa cgaggtcttg 120
   aagtttggag agttcaccct caagtccggc cgtatctccc cctacttcct gaacgcgggt 180
   ctcttcaaca ctggcgcttc gctctccaag atcggaaagt tctacgccgc tgccgtcaac 240
   gactcgggca ttgagcacga catcatcttt ggccccgctt acaagggtgt ccctcttgcc 300
   tgcaccaccg tcattgcctt ggccgaggcc ccctacaaca aggacacgcc ttactgcttc 360
   aaccgcaagg aaaagaagga ccatggcgag ggtggcacga ttgtcggatc ggcgttggag 420
   ggcaaggtcc tggtcattga cgatgttatc accgccggta ccgccatccg cgagtctgtt 480
   caaatcatcg aggactgcaa ggcccaattg gccggtgttt tggtggcggt ggatcgtcag 540
   gagactggca agaacggcga catgtctgct atccaggagg tcgagaggga tttcggtgtc 600
   cctgtcaagg ccattgtgac catgacccac atcatgcagt acatggagga gaagggtacc 660
   tatggcgagc acttgactca gatgcgcacc taccgcgaga agtacggtgt ttaaggcaaa 720
   tctaaatggg ataaggtccg gtataatgcg gcgagggaag gttctgttgg aaaatctcat 780
   aatgcgggga gattgacatc ggggaacgat gtgctgctca tcagcagttt tcgtggactc 840
   tcgggacagg ccttcctggg aatccagaca ataatcatta ataaatacca ataacaatca 900
   aaaaaaaaaa aaaaaaaaaa aaaaa 925
<210> 6
   <211> 217
   <212> PRT
   <213> Mortierella alpina
<400> 6
   Met Ala Ile Lys Asp Tyr Gln Arg Glu Phe Ile Glu Phe Ala Ile Lys
   1 5 10 15
   Asn Glu Val Leu Lys Phe Gly Glu Phe Thr Leu Lys Ser Gly Arg Ile
   20 25 30
   Ser Pro Tyr Phe Leu Asn Ala Gly Leu Phe Asn Thr Gly Ala Ser Leu
   35 40 45
   Ser Lys Ile Gly Lys Phe Tyr Ala Ala Ala Val Asn Asp Ser Gly Ile
   50 55 60
   Glu His Asp Ile Ile Phe Gly Pro Ala Tyr Lys Gly Val Pro Leu Ala
   65 70 75 80
   Cys Thr Thr Val Ile Ala Leu Ala Glu Ala Pro Tyr Asn Lys Asp Thr
   85 90 95
   Pro Tyr Cys Phe Asn Arg Lys Glu Lys Lys Asp His Gly Glu Gly Gly
   100 105 110
   Thr Ile Val Gly Ser Ala Leu Glu Gly Lys Val Leu Val Ile Asp Asp
   115 120 125
   Val Ile Thr Ala Gly Thr Ala Ile Arg Glu Ser Val Gln Ile Ile Glu
   130 135 140
   Asp Cys Lys Ala Gln Leu Ala Gly Val Leu Val Ala Val Asp Arg Gln
   145 150 155 160
   Glu Thr Gly Lys Asn Gly Asp Met Ser Ala Ile Gln Glu Val Glu Arg
   165 170 175
   Asp Phe Gly Val Pro Val Lys Ala Ile Val Thr Met Thr His Ile Met
   180 185 190
   Gln Tyr Met Glu Glu Lys Gly Thr Tyr Gly Glu His Leu Thr Gln Met
   195 200 205
   Arg Thr Tyr Arg Glu Lys Tyr Gly Val
   210 215
<210> 7
   <211> 30
   <212> DNA
   <213> Artificial Sequence
<220>
   <223> Description of Artificial Sequence : Artificially Synthesized Primer Sequence
<400> 7
   cttctcttca gcccccagcc gcatcggtcc 30
<210> 8
   <211> 30
   <212> DNA
   <213> Artificial Sequence
<220>
   <223> Description of Artificial Sequence : Artificially Synthesized Primer Sequence
<400> 8
   gagtccacga aaactgctga tgagcagcac 30
<210> 9
   <211> 20
   <212> DNA
   <213> Artificial Sequence
<220>
   <223> Description of Artificial Sequence : Artificially Synthesized Primer Sequence
<400> 9
   acaggtacac ttgtttagag 20
<210> 10
   <211> 20
   <212> DNA
   <213> Artificial Sequence
<220>
   <223> Description of Artificial Sequence : Artificially Synthesized Primer Sequence
<400> 10
   cgctgcgttc ttcatcgatg 20
<210> 11
   <211> 28
   <212> DNA
   <213> Artificial Sequence
<220>
   <223> Description of Artificial Sequence: Artificially Synthesized Primer Sequence
<400> 11
   ccatggatgg agtcgattgc gccattcc 28
<210> 12
   <211> 30
   <212> DNA
   <213> Artificial Sequence
<220>
   <223> Description of Artificial Sequence: Artificially Synthesized Primer Sequence
<400> 12
   ggatccttac tgcaacttcc ttgccttctc 30
<210> 13
   <211> 20
   <212> DNA
   <213> Artificial Sequence
<220>
   <223> Description of Artificial Sequence : Artificially Synthesized Primer Sequence
<400> 13
   ctttgtgggc atgcagatca 20
<210> 14
   <211> 22
   <212> DNA
   <213> Artificial Sequence
<220>
   <223> Description of Artificial Sequence : Artificially Synthesized Primer Sequence
<400> 14
   tgaagatgga gctgtggtgg ta 22